(19) Europäisches Patentamt
European Patent Office
Office européen des brevets

(11) Publication number : **0 366 171 B1**

(12) **EUROPEAN PATENT SPECIFICATION**

(45) Date of publication of patent specification : **06.05.92 Bulletin 92/19**

(51) Int. Cl.$^5$ : **C07H 19/06**, A61K 31/70

(21) Application number : **89202509.9**

(22) Date of filing : **05.10.89**

(54) **Nucleoside derivatives.**

Consolidated with 89911797.2/0437527 (European application No./publication No.) by decision dated 26.07.91.

(30) Priority : **05.10.88 GB 8823319**

(43) Date of publication of application : **02.05.90 Bulletin 90/18**

(45) Publication of the grant of the patent : **06.05.92 Bulletin 92/19**

(84) Designated Contracting States : **AT BE CH DE ES FR GB GR IT LI LU NL SE**

(56) References cited :
**F.E.B.S. LETTERS, vol. 234, July 1988, Amsterdam, NL; P.F. TORRENCE ET AL.: "Aids dementia: synthesis and properties of aderivative of 3'-azido-3'-deoxythymidine (AZT) that may become 'locked' in the central nervous system" pages 135-140**

(56) References cited :
**CHEMICAL ABSTRACTS, vol. 105, no. 22, 1st December 1986, abstract no. 197042f, Columbus, Ohio, US; K.H. RAND et al.:"Potential treatment of herpes simplex virus encephalitis by brain-specific delivery of trifluorothymidine using a dihydropyridinepyridinium salt type redox delivery system", & J. MED. VIROL. 1986, 20(1), 1-8**
**CHEMICAL ABSTRACTS, vol. 62, no. 7, 29th March 1965, abstract no. 8067e, Columbus, Ohio, US; G. Di SABATO et al.: "Thedenaturation of lactic dehydrogenases. II. The effect of urea and salts", & J. BIOL. CHEM. 240(3), 1072-6 (1965)**

(73) Proprietor : **NYCOMED AS**
**Nycoveien 1-2 Postboks 4220 Torshov**
**N-0401 Oslo 4 (NO)**

(72) Inventor : **Klaveness, Jo**
**Skoyen Terrasse 15**
**N-0276 Oslo 2 (NO)**
Inventor : **Rise, Frode**
**Östra Förstadsgatan 5a**
**S-21131 Malmö (SE)**
Inventor : **Undheim, Kjell**
**Evjeveien 32**
**N-1300 Sandvika (NO)**

(74) Representative : **Holmes, Michael John et al**
**Frank B. Dehn & Co. European Patent Attorneys Imperial House 15-19 Kingsway**
**London, WC2B 6UZ, (GB)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Jouve, 18, rue Saint-Denis, 75001 PARIS

## Description

This invention relates to antiviral compounds and more particularly to N-alkyldihydronicotinyl derivatives of nucleoside derivatives which are active against human immunodeficiency virus (HIV), the retrovirus which causes the disease AIDS.

AIDS is a relatively new disease. It was discovered in 1981 and several thousand cases of the disease have been diagnosed since then. It is anticipated that the number will increase to at least several hundred thousand in the next few years. The situation is especially severe in several Central African countries. AIDS is fatal, and about 40% of all diagnosed cases have ended in death. Of those diagnosed as having AIDS three or more years ago it is estimated that 85% are now dead.

Clinical symptoms are weight loss, chronic diarrhoea, persisting fever and opportunistic infections due to loss of T-cells, thus upsetting the overall balance of the immune system. The patient loses his/her ability to combat otherwise insignificant infections.

Several different methods to combat the infection have been tried. Among the methods tried are stimulation of the immune system and conventional treatment of the (secondary) life-threatening infections. So far the most promising method has been to attack the replication of the HIV-virus. Several different compounds interfering with replication have been tried, e.g. phosphonoformate (Foscarnet), suramin, Evans Blue, 3′-azido-3′-deoxythymidine (AZT) and 2′, 3′-dideoxynucleosides.

European Patent Application No. 0196185A, for instance, describes pharmaceutical compositions containing AZT, a known compound which has shown great promise in the treatment of AIDS and AIDS-related complex. It is believed that AZT works by inhibiting reverse transcriptase, a vital enzyme in the life cycle of retroviruses.

Further work has been done on alternative reverse transcriptase inhibitors which might avoid the limitations and drawbacks of AZT, for instance bone marrow suppression or the need for frequent administration of relatively large quantities, and among those suggested have been the 2′,3′-dideoxynucleosides.

The synthesis and activity of these compounds have been described (Mitsuya and Broder, Proc. Natl. Acad. Sci. 83, 1911 (1986)) and it was demonstrated that both the 2′ and 3′ positions must be unsubstituted while the 5′-hydroxy group must be present, presumably to allow in vivo conversion to the corresponding nucleotides.

European Patent Application No 0206497A discloses 2′,3′-dideoxyribofuranoside derivatives of cytosine or purine bases as antiviral compounds. While there is reference to esters of these compounds as possible metabolic precursors, there is no suggestion that esters would possess any advantageous properties compared with the parent 5′-hydroxy compounds and no esters are specifically named or their synthesis exemplified. There is no reference to any corresponding thymidine compounds or of any nucleoside derivatives having N-acylated amino groups.

We have now found that esterification or etherification of the 4- or the 5′- oxygen and/or amidation of exocylic or endocyclic nitrogen atoms present in the purine or pyrimidine ring can give significant advantages in terms of uptake, overall activity and site of action when at least one of the ester, ether or amide groups includes an N-alkyldihydronicotinyl group. Our copending PCT Application PCT/GB88/00224 describes certain esters and amides of this type carrying acyl groups at the 5′-position or on exocyclic nitrogens; the present invention extends this principle to a wider range of related compounds.

Thus according to one feature of the invention we provide compounds of formula (I)

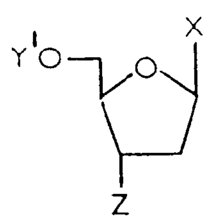

(I)

(wherein Z is a hydrogen atom or an azido group,

$Y^1$ is a hydrogen atom or a physiologically acceptable group of the formula

$$R^1(O)_nCO(OCR^2R^3)_m-$$

where n is 0 or 1, m is 0 or 1 and

$R^1$ is an optionally substituted alkyl or aryl group or, where n is 0, a hydrogen atom.

$R^2$ and $R^3$ are independently hydrogen atoms or lower alkyl groups; and

X is a group selected from

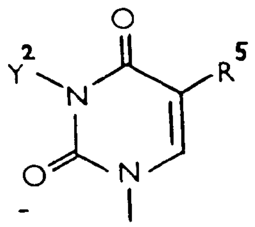

(A)

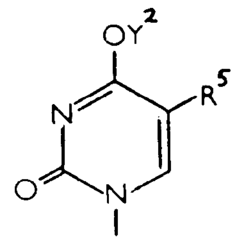

(B)

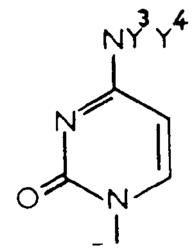

(C)

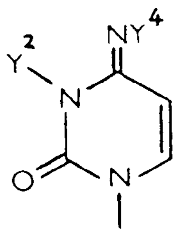

(D)

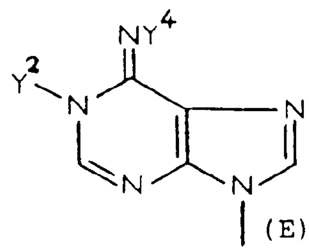

(E)

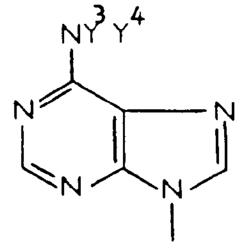

(F)

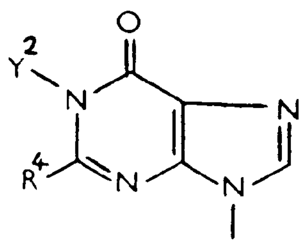

(G)

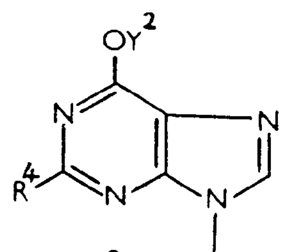

(H)

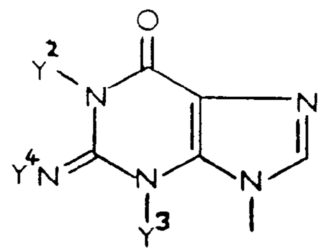

(I)

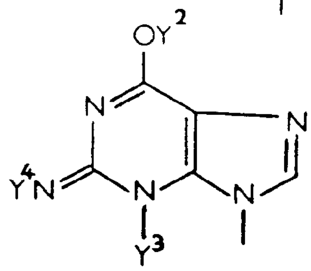

(J)

where the groups $Y^2$, $Y^3$ and $Y^4$ are as defined for $Y^1$ and may be the same as or different from $Y^1$ or each other, $R^4$ is a hydrogen atom or a group $-NY^3Y^4$, where $Y^3$ and $Y^4$ have the above meanings and $R^5$ is a hydrogen atom or a lower alkyl group, with the proviso that at least one of the groups $Y^1$, $Y^2$, $Y^3$ and $Y^4$ is a group of formula (III)

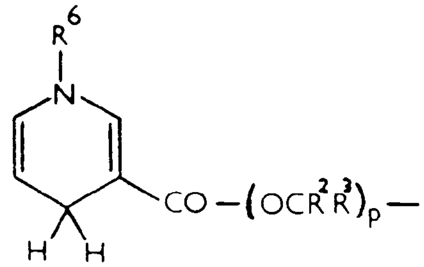

(III)

wherein $R^6$ is a $C_{1-7}$ alkyl group or $C_{7-10}$ aralkyl group, $R^3$ and $R^3$ are as defined above and p is 0 or 1, and that when $Y^1$ is a group of formula (III) as defined above, Z is an azido group and X is a group of formula (A) or (B) as defined above then $Y^2$ is a group of formula (III) or of formula $R^1(O)_nCO(OCR^3R^4)_m$- as defined above.

According to a further feature of the invention we provide for the use of compounds of formula (I) as hereinbefore defined, and/or salts thereof, in the manufacture of a composition for the treatment or prophylaxis of retrovirus infections, in particular neurotropic viruses and especially HIV infections. Such compositions also form part of the invention.

The group $R^1$ is preferably an alkyl group containing 1-20 carbon atoms which may be straight or branched, or an aryl group R which may contain 6 to 20 carbon atoms and may be mono- or poly-cyclic. Substituents which may be present on the alkyl groups $R^1$ include aryl groups preferably having 6-10 carbon atoms (as in aralkyl groupings), hydroxy and carboxy groups. Aryl groups include heterocyclic aryl groups such as pyridinyl and thienyl groups. Substituents which may be present on aryl groups include alkyl groups, e.g. having 1-6 carbon atoms, hydroxy and carboxy groups. Examples of such groups include methyl, ethyl, propyl, t-butyl, pentyl, stearyl, palmityl, carboxyethyl and benzyl groups.

The lower alkyl groups $R^2$, $R^3$ and $R^5$ preferably contain 1-6 carbon atoms. However, $R^2$ preferably represents a hydrogen atom, $R^3$ is preferably hydrogen or more preferably a methyl group. $R^5$ is preferably a hydrogen atom or a methyl group. $R^6$ is preferably a methyl group.

It will be noted that the compounds of the invention may carry more than one of the groups $Y^1$, $Y^2$, $Y^3$ and $Y^4$ and indeed it is preferred that in addition to the group of formula III there is at least one group of formula $R(O)_nCO(OCR^2R^3)$-in order to assist penetration of the blood-brain barrier as discussed below. In the compounds of formula (I) D, E, I and J, it is preferred that m in the group $Y^4$ is zero.

Groups $Y^2$ are preferably of the formula $R^1$.CO-, $R^1$CO.O.$CR^2R^3$ or $R^1$.O.CO.O.$CR^2R^3$-.

The salts of the compounds of formula (I) may be acid addition salts with organic or inorganic acids, for instance hydrochloric or phosphoric acid or methanesulphonic acid, ethane disulphonic acid, 2-naphthylsulphonic acid, pivalic acid and pamoic acid. Antiviral counter-ions such as phosphonoformate or suramin may also be used. Organic or inorganic base salts may be formed with acidic groups present in the molecule; suitable counter-ions include alkali metal ions such as sodium and potassium ions, divalent ions such as calcium and zinc ions and organic ions such as tetraalkylammonium and choline or ions derived from meglumine or ethylenediamine. Salts according to the invention may be formed by reaction of the compound of formula (I) with an appropriate acid or base.

The compositions according to the invention may be used in the treatment and/or prophylaxis of retrovirus infections, in particular HIV infections, and such a method forms a further feature of the invention. They may be formulated in conventional manner by admixture of one or more compounds of formula (I) as defined above with excipients and/or carriers.

It is believed that the compounds of formula (I) are not themselves inhibitors of reverse transriptase but are converted in vivo to the corresponding 2′,3′-dideoxy or 3′-azido-2′,3′-dideoxynucleosides. Nevertheless the derivatization with N-alkyldihydronicotinic acid, alone or in combination with the other acylations and alkylations according to the invention, gives surprising advantages in terms of uptake and sustained activity. The compounds of formula (I) are more lipophilic than the parent compounds and this permits rapid and efficient absorption from the gastro-intestinal tract; the absorption rate may be optimised by careful choice of the substituent group to give the desired balance of lipophilicity and hydrophilicity. The lipophilic nature of the compunds of

formula (I) also gives the molecules the ability to penetrate the cell membranes more easily and leads to higher intracellular concentrations, giving an improved dose/effect ratio. The steady hydrolysis of the compounds ensures a sustained concentration of the active compound in the cell and thereby permits longer intervals between doses, overcoming a significant drawback of the prior art compounds such as AZT.

Finally, the compounds according to the invention can penetrate the blood-brain barrier and thus permit treatment of the neurological disorders which have been observed to be related to the presence of neurotropic viruses, e.g. retroviruses such as HIV, and lentiviruses (Yarchoan et al, The Lancet, January 17, 1987, page 132). This is a significant advantage compared to the corresponding unsubstituted compounds or other antiviral compounds and is not referred to anywhere in the prior art, for instance in EP-A-0196185 or in EP-A-0206497. Attempts have been made to treat these neurological disorders with AZT but with limited success.

Bodor et al (Science, vol 214, December 18, 1981, pp - 1370-72) and International Patent Application WO83/03968 disclose the use of dihydropyridinyl/ pyridinium redox carriers for drugs which are required to pass the blood - brain or blood-testis barriers.

Thus, as disclosed in Bodor et al, loc cit, the N-alkyldihydronicotinyl group is oxidised in vivo to yield an N-alkylnicotinyl group, that is a group containing a quaternary nitrogen atom and therefore carrying a positive charge. While the reduced, uncharged molecule can penetrate the blood-brain barrier with great ease, once oxidised the charged molecule is trapped within the brain and a significant concentration of oxidised carrier-linked drug can then be maintained within the brain. The drug itself is liberated within the brain by slow ester hydrolysis.

Bodor does not disclose the application of the above redox system to any of the antiviral nucleosides concerned in European Patent Application No. 020649A. It will be appreciated that it is important that the rate of hydroylsis to liberate the free nucleoside should be relatively slow in order to provide a reservoir of the quaternised form of the drug trapped inside the blood-brain or blood-testis barrier. Such rates of hydrolysis vary from compound to compound and it was not obvious that the above redox system would be beneficial in such nucleosides. The disclosure of Bodor does not suggest attachment of redox systems such as N-alkyl-dihydronicotinyloxy groups to the heterocyclic bases in nucleosides or attachment of such a group to a nucleoside oxygen or nitrogen via a methylene or substituted methylene group.

Torrence et al (FEBS Letters, vol 234, July 1988, pp 135-140) disclose the preparation of the N-methyl-dihydronicotinyl derivative of AZT. However this derivative is only acylated on the 5′-position of the sugar residue, and there is no suggestion of acylation of the nitrogen or oxygen atoms in the base residue. There is also no suggestion of multiple acylation, e.g. the combination of an N-alkyl-dihydronicotinyl group and a second acyl group or of two N-alkyl-dihydronicotinyl groups to ensure good penetration of the blood-brain barrier.

The invention thus further provides a method of treatment of neurological disorders caused by neurotropic viruses wherein an effective dose of a compound of formula (I) or a salt thereof is administered to a patient suffering from such a disorder.

Compounds of formula (I) may be prepared in any convenient way, for example, by reaction of a compound of formula (II)

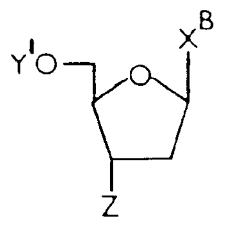

(II)

[wherein $Y^1$ and Z are as hereinbefore defined and $X^B$ is as hereinbefore defined for X except that any of the groups $Y^1$, $Y^2$, $Y^3$ and $Y^4$ may each additionaly represent a protecting group, with the proviso that at least one of $Y^1$, $Y^2$, $Y^3$ and $Y^4$ is a hydrogen atom] with a reagent serving to introduce a group $R^1(0)_n.CO.(OCR^2R^3)_m$- followed where required by removal of any protecting groups and/or unwanted substituents so introduced.

It should be noted that where, in the starting material, more than one of $Y^1$, $Y^2$, $Y^3$ and $Y^4$ is hydrogen, multiple reactions may occur.

Where it is desired to ensure that acylation or alkylation is effected while one or more groups $Y^1$, $Y^2$, $Y^3$ and $Y^4$ remain as hydrogen atoms, it may be desirable to protect the latter first, to form a compound of formula (I) in which one or more of $Y^1$, $Y^2$, $Y^3$ and $Y^4$ are protecting groups, these being removed after introduction of the desired acyl or ether group. Such protecting groups may, in fact, be conventional N- or O-protecting groups including groups $R^1OCO$- which may be selectively removed in the presence of the group(s) intended to remain.

Thus, for example, an N-benzyloxycarbonyl may be used to protect an exocylic amino group and if the group which is intended to remain is not one which is removable by reduction, for example a straight chain alkoxycarbonyl group, the N-benzyloxycarbonyl group can readily be removed selectively using hydrogen and a noble metal catalyst such as palladium.

Trisubstituted silyl groups may also be used as protecting groups, especially for the 5′-oxygen atom, and include trialkylsilyl such as trimethylsilyl, thexyldimethyl-silyl and dimethyl-t-butylsilyl groups.

In general, where more than one of $Y^1$, $Y^2$, $Y^3$ and $Y^4$ are hydrogen, and a mixture of compounds is produced, the individual components may readily be separated, for example by chromatography.

Where 5′-O-monoalkylation is to be effected (i.e. introduction of a group $Y^1$ in which m is 1) it is especially effective to form a dianion of the nucleoside (e.g. by reacting with sodium hydride) and to react this with one equivalent of the alkylating agent. It is, of course, still possible to use protected forms of the nucleoside, for example by acylation of a nucleophilic nitrogen atom before salt formation with sodium hydride.

Suitable acylating agents for use in the reaction have the formula Ac-L where L is a leaving group. When the acyl group Ac- is derived from a carboxylic acid, i.e. is of formula $R^1$-CO-, then suitable acylating agents include the acid halides and acid anhydrides advantageously in the presence of a base; when the acyl group is derived from a carbonic acid, i.e. is of formula $R^1$.O.CO-, then acylating agents include the haloformate esters and reactive carbonic acid diesters. In such reagents, the halogen may, for example, be chlorine or bromine. The base for use in the reaction with the acid halide or anhydride may, for example, be a heterocyclic base such as pyridine or 4-dimethylaminopyridine. The latter increases the speed of the reaction and may be used advantageously with pyridine. The reaction will normally be carried out in the presence of an inert solvent e.g. a substituted amide solvent such as dimethylformamide, dimethylacetamide or a halogenated hydrocarbon such as dichloromethane.

In general, we have found that using acid anhydrides as acylating agents to introduce a group $R^1$CO, O-acylation at the 5′- position takes place more readily than N-acylation, whereas using acid halides, N-acylation or even N-diacylation predominates. However, N-acyl groups $R^1$CO- may be removed selectively, for example by reaction with a phenol such as p-methyl-phenol. Where multiple substitution is to be effected, a stronger base such as sodium hydride may be advantageous.

Suitable acyloxyalkylating agents for use in the invention will in general be of the formula $R^1$CO.O.C$R^2R^3$L or $R^1$O.CO.O.C$R^2R^3$L, where L is a leaving group. Thus, the group L may for example, be a halogen atom such as a chlorine or bromine atom or a hydrocarbon-sulphonyloxy group such as a tosyloxy or mesyloxy group.

Where the reaction serves to introduce a nicotinyloxyalkyl group, i.e. a precursor for a group of formula (III) as defined above in which p is 1, then it is advantageous to use a reagent Ac-L in which the nicotinyl group has been protected with a readily displaceable substituent to prevent alkylation of the pyridine nitrogen atom, thus preventing polymerisation of the reagent. One method involves using a nicotinyl derivative in which one or two halogen atoms (e.g. chlorine, bromine or iodine) are present in the position ortho to the nitrogen atom, such as 2-chloronicotinic acid or preferably 6-chloronicotinic acid. After conversion of this derivative to the alkylating form Ac-L and alkylation of the nucleoside, the substituent may be removed, in the case of chlorine for instance by hydrogenolysis over palladium/charcoal.

The alkylation reaction will normally be effected in the presence of a base, conveniently an inorganic carbonate such as potassium carbonate or an alkali metal hydride such as sodium hydride. Bases as used for acylation may also be useful.

The starting compounds of formula (II) wherein $Y^1$, $Y^2$, $Y^3$ and $Y^4$ are all hydrogen atoms are well described in the literature - see, for instance, Lin et al, J. Med. Chem. 30, 440 (1987). Starting compounds wherein one or more of $Y^1$, $Y^2$, $Y^3$ and $Y^4$ are other than hydrogen may be prepared by preliminary reactions as described above.

The compounds of formula (I) may also be prepared by reduction of the corresponding N-alkylnicotinyl analogues, which may in turn be prepared by acylation or etherification of a compound of formula (II) using reaction conditions as described above or by N-alkylation of the corresponding nicotinyl compound. Such alkylation may be achieved by reaction of a compound formula II in which at least one of $Y^1$, $Y^2$, $Y^3$ and $Y^4$ is hydrogen with a halomethyl nicotinic acid ester, e.g. the chloromethyl ester. The latter reagent may be obtained by reaction of an acid halide of nicotinic acid with formaldehyde, e.g. in the form of paraformaldehyde. Alternatively, the acid halide of nicotinic acid may be reacted with a mixture of paraformaldehyde and p-chlorothiophenol to give the p-chlorophenylthiomethyl ester of nicotinic acid which may then be reacted with sulphuryl chloride to give the desired chloromethyl ester.

Reduction of an N-alkyl nicotinyl compound to an N-alkyl dihydronicotinyl compound may be accomplished using a suitable reducing agent, such as sodium dithionite in an inert solvent such as diethyl ether or in a two phase solvent system such as diethyl ether/water.

N-Alkylation of a nicotinyl compound may be achieved with an alkylating agent such as an alkyl iodide, for

instance methyl iodide. Suitable solvents include alcohols, for instance methanol.

Acylation or alkylation to give the desired nicotinyl starting materials may be accomplished using an acylating or alkylating derivative of nicotinic acid as described above, for instance nicotinyl chloride hydrochloride or nicotinyloxymethyl chloride.

The pharmaceutical compositions according to the invention may be formulated conventionally by means well known in the art, and may be administered by any convenient route, for instance orally, rectally, vaginally, intraveneously or intramuscularly. Examples of suitable formulations include tablets and capsules, aqueous formulations for intravenous injection and oil-based formulations for intramuscular injection. Suitable dosages will lie in the range 0.1 to 100mg per kilogram of bodyweight per 24 hour period. The compositions according to the invention may also contain other active antivirals for instance acyclovir, phosphonoformate, suramin, Evans Blue, interferons or AZT. The invention is illustrated by the following Preparations and Examples (other starting materials are either known or are prepared according to our PCT Application PCT/GB88/00224). Capsugel is a Trade Mark.

Preparation 1

Chloromethyl 3-pyridinecarboxylate

Method A:

(Method based on literature - general method for making α-haloalkyl esters: M Neuenschwander et al; Helv. Chim. Acta 61(1978) 2047)

A mixture of 3-pyridinecarbonyl chloride hydrochloride (20 mmol) and anhydrous zinc chloride (10 mg) in dichloro-methane (25 ml) is stirred at ambient temperature under nitrogen for 30 min, the mixture cooled to 0°C and paraformaldehyde (25 m mol) added portionwise with vigorous stirring. The mixture is stirred at ambient temperature for 3 hours, the solvent distilled off and the residue subjected to a quick distillation at 0.005 mm Hg.

Method B:

Paraformaldehyde (25 m mol) is added portion-wise to the lithium salt of p-chlorothiophenol (20 m mol) in dry THF (30 ml) under nitrogen with stirring at 0°C, the mixture stirred at ambient temperature for 2 hours, 3-pyridinecarbonyl chloride hydrochloride (20 mmol) in dry THF (20 ml) added dropwise with stirring and the mixture stirred for 1 hour before the solvent is removed at reduced pressure. The residue is extracted with dichloromethane (3x25 ml), the solution containing the product, p-chlorophenylthiomethyl 3-pyridinecarboxylate, is washed with water, dried ($MgSO_4$) and sulphuryl chloride (20 m mol) in dry dichloromethane (20 ml) added drop-wise during 5 min with stirring at 0°C. The mixture is stirred for 10 min at ambient temperature and cyclohexene (20 m mol) in dry dichloromethane (20 ml) is added drop-wise during 10 min to trap the sulphenyl chloride as the corresponding adduct which has a high boiling point. The solvent is then distilled off and the chloromethyl ester isolated by a quick distillation of the residue as described above.

Preparation 2

$N^4$-Propinoyl-2′,3′-dideoxy-cytidine and $N^4$,5′-Q-dipropionyl-2′,3′-dideoxy-cytidine

$2^1,3^1$-Dideoxycytidine (0.1000g, $4.734.10^{-4}$ mole) and N,N-dimethylaminopyridine (0.0636g, $5.208.10^{-4}$ mole) were stirred in a mixture of dichloromethane (5ml) and dimethylformamide (1ml) at 0°C. Propionyl chloride (0.0438g, $4.74.10^{-4}$ mole) was added slowly (10min) with a gas tight syringe. The resulting mixture was stirred at room temperature for 24 hours. Water (1ml) was added. Water and organic solvents were removed by high vacuum evaporation. The products were purified by chromatography on a silica column with chloroform and chloroform: ethanol 9:1 as eluents.

$N^4$,5′-O-dipropionyl-2′,3′-dideoxy-cytidine
Yield: 0.0741 g (48.4%).
$N^4$-Propionyl-2′,3′-dideoxy-cytidine.
Yield: 0,035g (27.7%). White crystalline material. $^1$HNMR ($CDCl_3$, 300 MHz) δ : 1.22 (t,3H), 1.90 - 2.10 (m,1H), 2.15 - 2.26 (m,1H), 2.48 - 2.62 (m+q (AB), 1H [H2′ or H3′] and $CH_2$), 3.81 and 4.10 (d(AB), 2H, H5′), 4.25 - 4.33 (m, 1H, H4′), 6.12 (dd, 1H, H1′), 7.47 (d,1H′, H5), 8.49 (d,1H,H6), 9.20 (broad s, 1H, NH). $^{13}$CNMR($CDCl_3$, 75 MHz) δ: 8.45, 24.13, 30.71, 33.33, 62.99, 82.75, 88.28, 96.01, 145.31, 155.47, 162.35 and 174.24.

EXAMPLE 1

a) 2′,3′-Dideoxy-N⁴, 5′-0 di(pyridine-3-carbonyl)cytidine (Formula (I) C, Z= $Y^4$=H, $Y^3$ = $Y^1$ = (1,4-dihydro-N-methyl pyridin-3-yl carbonyl)

2′,3′-Dideoxycytidine (0.100 g, 5.12x$10^{-4}$ mol) and 4-N,N-dimethylaminopyridine (0.073 g, 6x$10^{-4}$ mol) are dissolved in a mixture of dichloromethane (5 ml, distilled from calcium hydride) and pyridine (5 ml) and cooled to 0°C. Pyridine-3-carbonyl chloride hydrochloride (1.126 m mol) is added gradually during 30 minutes. The resulting mixture is stirred at ambient temperature for 48 hours under nitrogen. Water (4 ml) is added and the solution is evaporated under reduced pressure. The product is purified by column chromatography (silica gel with chloroform and ethanol as eluents).

b) 2′,3′-Dideoxy-$N^4$,5′-0-bis(1-methyl-pyridinium-3-carbonyl)cytidine diiodide

2′,3′-Dideoxy-$N^4$, 5′-0-bis(pyridine-3-carbonyl cytidine (0.100 g 2.47x$10^{-4}$ mole) is dissolved in methanol (5 ml) and cooled to 0°C. Methyl iodide (0.07 g, 4.93x$10^{-4}$ mol) is added and the temperature is allowed to reach room temperature. The mixture is stirred for 2 hours at this temperature and is then refluxed for a short period. The solvent is removed and the product is isolated by crystallization.

c) 2′,3′-Dideoxy-$N^4$, 5′0-bis(1,4-dihydro-1-methylpyridine-3-carbonyl) cytidine.

2′,3′-Dideoxy-$N^4$, 5′-0-bis(1-methylpyridinium-3-carbonyl)cytidine diiodide (0.100g, 1.06x$10^{-4}$ mol) is dissolved in deaerated water (5 ml) containing sodium bicarbonate (6.36x$10^{-4}$ mol). Diethyl ether (10 ml) is added and the resulting two phase system is cooled to 0°C with an ice bath. Sodium dithionite (4.24x$10^{-4}$ mol) is added during 10 minutes. The mixture is stirred under nitrogen for 12 hours. The organic layer is separated and the water phase is extracted with diethyl ether (3.25 ml). The combined ether phase is washed with water, dried with magnesium sulphate and evaporated. The product is isolated by chromatography.

Example 2

3-(1,4-Dihydro-1-methylpyridine-3-carbonyloxy)methyl - 5′ O-palmitoyl-2′,3′-dideoxythymidine (Formula (I)A, $Y^2$=1,4-dihydronicotinyl-oxymethyl, $Y^1$ = palmitoyl, $R^5$ = methyl)

(a) Chloromethyl N-methylpyridinium-3-carboxylate chloride

1-Methylpyridinium-3-carbonyl chloride (1 ml) is suspended in dioxan (50 ml), paraformaldehyde (1.5 mmol) added and the mixture stirred at ambient temperature overnight. The solvent is removed at reduced pressure and the crude product is used directly for the alkylation in the subsequent reaction.

b) A mixture of 5′O-palmitoyl-2′,3′-dideoxythymidine (1 mmol), chloromethyl N-methylpyridinium-3-carboxylate chloride (1 m mol) and potassium carbonate (0.5 m mol) in dry N,N-dimethylacetamide (50 ml) is stirred at ambient temperature overnight. The solvent is removed at reduced pressure, the residue triturated with a small volume of water, filtered, the filtrate washed with a little acetone and the solid dried. The pyridinium derivative of the nucleoside thus prepared is added to aqueous ethanol (1:1; 25 ml) containing sodium carbonate (0.8 mmol). Diethyl ether (25 ml) is added, the mixture cooled to 0°C, sodium dithionite (1 mmol) added during 15 minutes, the mixture stirred under nitrogen for 12 hours, the organic layer separated, the water phase extracted with ether (2x50 ml), the combined ether phases washed with water, dried ($MgSO_4$), evaporated and the product purified by flash chromatography using light petroleum: ethyl acetate.

Example 3

3-(1,4-Dihydro-1-methylpyridine-3-carbonyloxy)methyl-5′-O-palmitoyl-3′-deoxythymidine (Formula (I)A, $Y^2$ = 1,4-dihydro-1-methylpyridine-3-carbonyloxymethyl,$Y^1$ = palmitoyl, $R^5$ = methyl

(a) 5′-O-Palmitoyl-3-(pyridine-3-carbonyloxy)methyl-3′-deoxythymidine

A mixture of 5′-O-palmitoyl-3′-deoxythymidine (1 mmol) and potassium carbonate (0.8 mmol) in dry N,N-dimethylacetamide (20 ml) is stirred at ambient temperature for 2 hours before a solution of chloromethyl pyridine-3-carboxylate (1.2 m mol) in dry N,N-dimethylacetamide (10 ml) is added dropwise with stirring at 0°C

during 30 min. The resultant mixture is stirred at ambient temperature for 35 hours, filtered, the filtrate evaporated and the product purified by flash chromatography on silica gel using ethyl acetate: light petroleum.

b) 3-(1-Methylpyridinium-3-carbonyloxy)methyl-5′-O-palmitoyl-3′-deoxythymidine iodide

Methyl iodide (2 mmol) is added to a solution of 5′-0-palmitoyl-3-(pyridine-3-carbonyloxy)methyl-3′-deoxythymidine (1 mmol) in anhydrous acetone (20 ml), the flask containing the solution wrapped in aluminium foil, the solution stirred at ambient temperature for 48 hours before the precipitated product is isolated by filtration.

c) 3 (1,4-Dihydro-1-methylpyridine-3-carbonyloxy)methyl-5′-0-palmitoyl-3′-deoxythymidine

3-(1-Methylpyridinium-3-carbonyloxy)methyl-5′-0-palmitoyl-3′-deoxythymidine iodide (1 mmol) is dissolved in deaerated 10% aqueous methanol (75 ml), the solution cooled to 0°C, sodium bicarbonate (4 mmol) and sodium dithionite (4 mmol) added, the resultant mixture stirred at ambient temperature for 20 min before the precipitated yellowish product is removed by filtration and is washed with water and dried. The product can be further purified by flash chromatagraphy on silica gel using ethyl acetate:light petroleum.

Example 4

i) Phenylthiomethyl 6-chloropyridine-3-carboxylate

Sodium hydride (0.315 g, 21 mmol) was added portion wise to a solution of 6-chloronicotinic acid (11.03 g, 70 mmol) in 1,2-dimethoxyethane (250 ml) at 4°C, the mixture heated to 40°C, chloromethyl phenyl sulfide (11.10 g, 70 mmol), sodium iodide (0.315 g, 2.1 mmol) and tetrabutylammonium bromide (1.35 g, 4.2 mmol) added. The mixture was heated under reflux ($N_2$-atmosphere) and exclusion of light for 20 hours when TLC monitoring showed the reaction to be complete. The cold mixture was filtered, the filtrate evaporated,the residue dissolved in dichloromethane (10 ml), the solution washed with saturated NaCl-water and 2N sodium carbonate, dried ($MgSO_4$), evaporated and the residue distilled; b.p. 135°C/0.005 mmHg, yield 80% (0.97 g), m.p. 142-144°C. $^1$H NMR ($CDCl_3$): δ 5.67 ($CH_2$, s), 7.2 - 7.7 (Ph,H-5), 8.49 (H-4, dd, J 2.5, 8.5 Hz), 8.97 (H-2, d, J 2.5 Hz).

ii) Chloromethyl 6-chloropyridine-3-carboxylate

Sulfuryl chloride (1.89 g, 14 mmol) in dry dichloromethane (15 ml) was added dropwise during 30 minutes to a stirred solution of phenylthiomethyl 6-chloropyridine-3-carboxylate (2.80 g, 10 mmol) in dry dichloromethane (30 ml) at 0°C and under $N_2$. The mixture was stirred for another 30 minutes at 0°C, at room temperature for 60 minutes, cooled to 0°C and a solution of cyclohexene (0.90 g, 11 mmol) in dry dichloromethane (10 ml) added dropwise during 10 minutes. The resultant mixture was stirred at room temperature for 2.5 hours, evaporated, light petroleum added, the precipitate removed by filtration through a bed of silica gel and the filtrate chromatographed on a silica column using gradient elution:$CH_2Cl_2$: light petroleum (2:3) to $CH_2Cl_2$:EtOAc(9:1); yield 95% (1.97 g). $^1$H NMR($CDCl_3$)δ:5.92 ($CH_2$), 7.43 (H-5,d, J 8.5 Hz), 8.27 (H-4, dd, J 2.5,8.5 Hz), 9.03 (H-2, d, J 2.5).

iii) 5′-O-(6-Chloropyridine-3-carbonyloxy)methyl-3′-deoxythymidine

3′-Deoxythymidine (0.5 mmol) is dissolved in dry DMF (10ml), sodium hydride (1.13 mmol) added, the mixture stirred at room temperature for 20 minutes, cooled to -40°C and a solution of chloromethyl 6-chloropyridine-3-carboxylate (0.53 mmol) in DMF (5 ml) added dropwise with stirring. The reaction is monitored by TLC. After 30 minutes at -40°C the mixture is allowed to reach room temperature, stirred at this temperature for 2 hours, filtered, the filtrate evaporated at reduced pressure and the filtrate subjected to flash chromatography using $CHCl_3$:EtOH (9:1).

iv) 5′-O-(Pyridine-3-carbonyloxy)methyl-3′-deoxythymidine

Magnesium oxide (2 mmol) and 5% Pd on charcoal (65 mg) are added to a solution of 5′-O-(6-chloropyridine-3-carbonyloxy)methyl-3′-deoxythymidine (0.5 mmol in DMF (4 ml) and ethanol (1 ml). The hydrogenolysis is run at atmospheric pressure using a Brown apparatus where the $H_2$-gas is generated under controlled conditions by the addition of 3 N HCl to an aqueous solution of sodium borohydride. The reaction is run for 2 hours at 50°C (TLC monitoring). The mixture is then filtered through a bed of Celite and the filtrate taken to dryness under reduced pressure. The product is further purified by flash chromatography on silica gel using $CHCl_3$:EtOH(9:1).

v) 5′-O-(1-Methylpyridinium-3-carbonyloxy)methyl-3′-deoxythymidine iodide

Methyl iodide (2 ml) is added to a solution of 5′-O-(pyridine-3-carbonyloxy)methyl-3′-deoxythymidine (1 mmol) in acetone (20 ml), the mixture stirred at room temperature for 48 hours under the exclusion of light, and the precipitated product is isolated by filtration.

vi) 5′-O-(1,4-Dihydro-1-methylpyridine- 3-carbonyloxy)methyl-3′-deoxythymidine

5′-O-(1-Methylpyridinium-3-carbonyloxy)methyl-3′-dideoxythymidine (1 mmol) is dissolved in deareated 10% aqueous methanol (75 ml), the solution cooled to 0°C, sodium bicarbonate (4 ml) and sodium dithionite (4 mmol) added, the resultant mixture stirred at room temperature for 20 minutes before the precipitated product is removed by filtration, washed with water and dried. The product can be further purified by flash chromatography on silica gel using $CHCl_3$:MeOH(9:1).

Example 5

i) 5′-O-(3″-Pyridinylcarbonyl)-N4-propionyl-2′,3′-dideoxy-cytidine

$N^4$-propionyl-2′,3′-dideoxycytidine (0.035g, $1.309.10^{-4}$-mole) and N,N-dimethylaminopyridine (0.0329 g, $1.440.10^{-4}$ mole) were stirred in dry dichloromethane (2ml) at 0°C. Nicotinic anhydride (prepared according to Schrecher et al.J.Am,Chem.Soc, 5803 (1954) from sodium nicotinate) (0.0178 g, $1.453.10^{-4}$ mole) was stirred at room temperature for 12 hours, more nicotinic anhydride (0.0178g, $1.453.10^{-4}$ mole) was added and the mixture was stirred for 12 more hours. Water (2ml) was added. Water and dichloromethane were removed by high vacuum evaporation. The product was purified by chromatography on a silica column with chloroform and chloroform:ethanol 9:1 as eluants.

Yield: 0.0278g (57.1%) (approximately 10% more in impure fractions (oil). $^1$HNMR ($CDCl_3$ 300 MHz) δ: 1.72 (t,$CH_3$), 1.75 - 1.83 (m, 1H), 2.10 - 2.22 (m, 2H) 2.49 (q, $CH_2$), 2.50 - 2.66 (m,1H), 4.44 4.54 (m, 1H, H4′), 4.62 (broad d, 2H, H5′), 6.04 (dd, 1H, H1′), 7.35 (d, 1H, H5), 7.42 (dd, 1H, H5′), 8.09 (d,1H, H6), 8.26 (dt, 1H, H4″), 8.79 (dd, 1H, H6″), 9.21 (t, 1H, H2″), 9.41 (broad s, 1H, NH).

$^{13}$CNMR($CDCl_3$ 75 MHz)δ : 8.69, 25.32, 30.,62, 39.09, 65.54, 79.64, 88.30, 96.06, 123.49, 123.43, 137.03, 143.64, 150.90, 153.96, 155.09, 162.61, 165.03, 174.21.

ii) 5′-O-(1″-Methyl-3″-pyridinylcarbonyl-N4-propionyl-2′,3′-dideoxycytidine Iodide

5′-O-(3″-Pyridinylcarbonyl)-$N^4$-propionyl-2′,3′-dideoxycytidine (0.0278g, $7.47.10^{-5}$ mole) was dissolved in acetone (4 ml) and methyl iodide (50μl) was added. The reaction mixture was gently refluxed and the reaction followed by TLC. More methyl iodide was added with 6 hour intervals (3x 50μl). The reaction mixture was evaporated after 24 hours reflux and the yellow product was dissolved in deuterated acetone and heavy water and analysed by $^1$HNMR.

Yield: aproximately 90% as seen by $^1$HNMR.

$^1$HNMR ($D_2O$/Acetone-$d_6$, 300 MHz)δ:1.00 (t,$CH_3$), 1.72 - 1.88 (m,1H), 2.822.18 (m, 2H) 2.34 - 2.44 (q, $CH_2$) 2.40 - 2.52 (m, 1H), 4.41 (s,N-$CH_3$), 5.95 (dd′, 1H, H4′), 7.17 (d, 1H, H5), 8.15 (dtt, 2H, H6-H5″), 8.95 (dd, 2H,H4″ and H6″), 9.40 (broad s, 1H, H2″), H4′ and H5 protons obscured by water resonance.

iii) 5′-O-(1″,4″-Dihydro-1″-methyl-3″-pyridinylcarbonyl)-N4-propionyl-2′,3′-dideoxycytidine

The product from (ii) (7.47 x $10^{-5}$ moles) was washed with a small amount of $CHCl_3$ to remove hydrophobic impurities and then dissolved in 10% aqueous methanol (3 ml). Sodium bicarbonate (0.031g; 3.735 x $10^{-4}$ moles) and sodium dithionite (0.065g; 3.735 x $10^{-4}$ moles) were added gradually over a period of 10 minutes. The reaction was run under an atmosphere of $N_2$ for one hour. The reaction mixture was evaporated under reduced pressure and the resulting residue aplied to a silica column and eluted with $CHCl_3$ and then $CHCl_3$/EtOH 9:1. The fractions containing the product were evaporated to yield an oil.

$^1$HNMR ($CDCl_3$, 300 MHz)δ : 1.17 (t,3H,Me), 1.9 (m,1H), 2.1 - 2.3 (m,2H), 2.4 - 2.65 (q+m,3H), 2.9 (s,3H,Me),

3.1 (broad s,2H), 4.5 (m,1H,H4′), 4.6 (m,2H,H5′), 4.8 (1H), 5.6 (1H), 6.0 (m,1H,H1′), 7.0 (1H), 7.4(d,1H,H5), 8.1 (d,1H,H6).

Pharmaceutical Example A

Preparation of capsules for oral use

| | |
|---|---|
| Active Compound | 50 mg |
| Amylum maydis | q.s. |

The powder is mixed and filled into hard gelatin capsules (Capsugel Size 00).

Pharamceutical Example B

Preparation of an ointment

| | |
|---|---|
| Active compound | 1 g |
| Liquid paraffin | 100 g |
| White soft paraffin | to 1000 g |

White soft paraffin is melted and incorporated into the liquid paraffin and stirred until the mixture is cold. Active compound is triturated with a portion of the basis and gradually the remainder of the basis was incorporated. The ointment is filled into lacquered aluminium tubes (20 g) and sealed. The ointment contains 0.1 % active compound.

Pharmaceutical Example C

Suspension for parenteral administration

| | |
|---|---|
| Active Coumpound | 200 gr |
| Polysorbate 80 | 3 gr |
| Sorbitol | 400 gr |
| Benzyl alcohol | 8 gr |
| Water | ad 1000 ml |
| 1M HCl | q.s. |

Polysorbate 80, Sorbitol and benzyl alcohol are dissolved in 500 ml distilled water. Active compound is screened through a 0.15 mm sieve and dispersed in the solution under vigorous stirring. The pH is adjusted to 4.5 by dropwise addition of 1M HCl. Water is added to 1000 ml, the suspension was filled in 1 ml vials The vials are sterilized by $\gamma$ -radiation. Each vial contains 200 mg active compound.

Pharmaceutical Example D

Preparation of tablets

| | Gram |
|---|---|
| Active Compound | 200 |
| Lactose | 85 |
| Polyvinylpyrrolidone | 5 |
| Starch | 42 |
| Talcum powder | 15 |
| Magnesium stearate | 3 |

Active compound and lactose are screened through a 0.15 mm sieve and mixed together for 10 minutes.

The mixed powder is wetted with an aqueous solution of polyvinyl-pyrrolidone. The mass is granulated, and the dried (40 °C) granulate is mixed with starch, talcum powder and magnesium stearate. The granulate is compressed into tablets. The tablet diameter is 11 mm, the tablet weight is 350 mg and each tablet contains 200 mg active compound.

Pharmaceutical Example E

Preparation of a suspension for rectal administration

Methyl parahydroxybenzoate (70 mg) and propyl parahydroxybenzoate (15 mg) are dissolved in water (100 ml) at 90 °C. After cooling to 30 °C methyl cellulose (2g) is added and the mixture is agitated for 3 hours. 1 gram active compound are screened through a 0.15 mm sieve, and dispersed in the solution under vigorous stirring. The suspension is filled in a 100 ml tube. The suspension contain 10 mg active compound/ml.

Pharmaceutical Example F

Preparation of oral suspension

| | Gram |
|---|---|
| Active Compound | 10 |
| Carboxymethyl cellulose | 1.5 |
| Sorbitol | 200 |
| Sodium benzoate | 1.0 |
| Orange essence | 0.3 |
| Apricot essence | 0.7 |
| Ethanol | 50 |
| Water | 236.5 |

Carboxymethyl cellulose, sorbitol and sodium benzoate are dissolved in water with stirring for 2 hours. A solution of the essences in ethanol is added. Active compound is screened through a 0.15 mm sieve and dispersed in the solution under vigorous stirring. The suspension (10 gram) is filled in a 20 ml tube. Each tube contains 200 mg active compound.

Pharmaceutical Example G

Preparation of injection solution

10 mg active compound are dissolved in 10 ml 0.9 % sodium chloride. pH is adjusted to 4.5 with 1N HCl. The solution is sterile filtered and filled into a 10 ml vial. The solution contains 1 mg active compound/ml.

Pharmaceutical Example H

Preparation of tablets (controlled release formulation)

| | Gram |
|---|---|
| Active Compound | 500 |
| Hydroxypropylmethylcellulose (Methocel K15) | 120 |
| Lactose | 45 |
| Povidone | 30 |
| Magnesium stearate | 5 |

Active compound, hydroxypropyl-methylcellulose and lactose are mixed together for 20 minutes and granulated with a solution of povidone. Magnesium stearate is added and the mixture is compressed into tablets. The tablet diameter is 13 mm, the tablet weight is 700 mg and each tablet contains 500 mg active compound.

## Claims

**Claims for the following Contracting States : AT, BE, CH, DE, FR, GB, IT, LI, LU, NL, SE**

1. Compounds of formula (I)

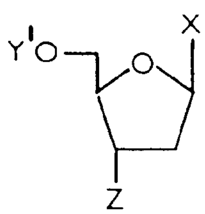

(I)

wherein Z is a hydrogen atom or an azido group,

$Y^1$ is a hydrogen atom or a physiologically acceptable group of the formula

$$R^1(O)_nCO(OCR^2R^3)_m-$$

where n is 0 or 1, m is 0 or 1 and

$R^1$ is an optionally substituted alkyl or aryl group or, where n is 0, a hydrogen atom;

$R^2$ and $R^3$ are independently hydrogen atoms or lower alkyl groups; and

X is a group selected from

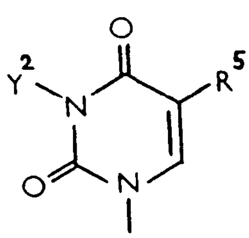

(A)

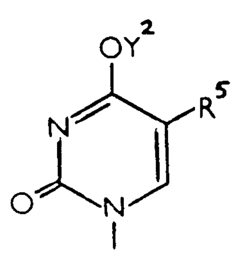

(B)

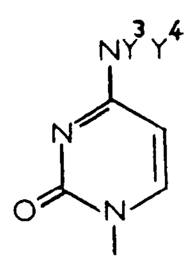

(C)

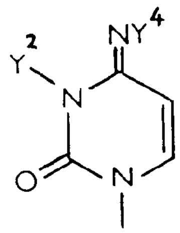

(D)

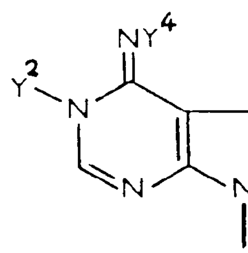

(E)

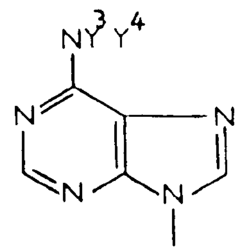

(F)

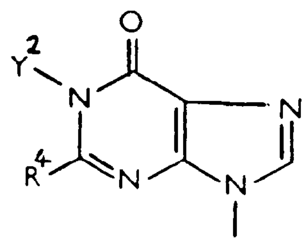

(G)

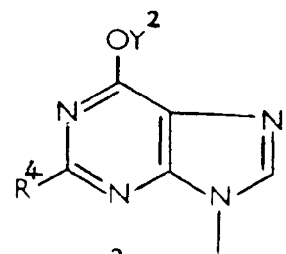

(H)

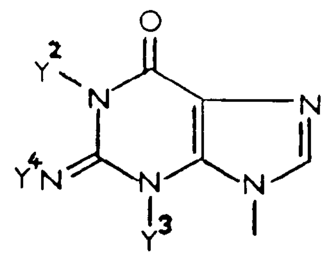

(I)

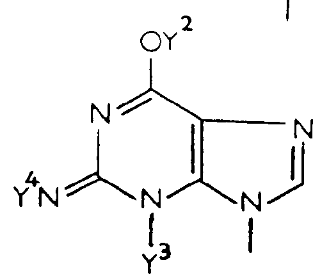

(J)

where the groups $Y^2$, $Y^3$ and $Y^4$ are as defined for $Y^1$ and may be the same as or different from $Y^1$ or each other, $R^4$ is a hydrogen atom or a group $-NY^3Y^4$, where $Y^3$ and $Y^4$ have the above meanings and $R^5$ is a hydrogen atom or a lower alkyl group, with the proviso that at least one of the groups $Y^1$, $Y^2$, $Y^3$ and $Y^4$ is a group of formula (III)

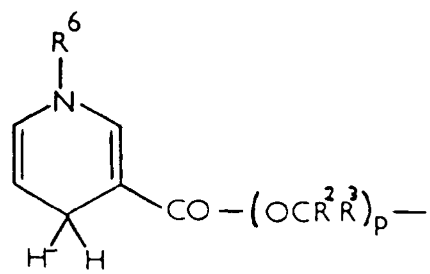

(III)

wherein $R^6$ is a $C_{1-7}$ alkyl group or $C_{7-10}$ aralkyl group, $R^2$ and $R^3$ are as defined above and p is 0 or 1, and that when $Y^1$ is a group of formula (III) as defined above, Z is an azide group and X is a group of formula (A) or (B) as defined above then $Y^2$ is a group of formula (III) or of formula $R^1(O)_nCO(OCR^3R^4)_m$- as defined above.

2. Compounds of formula (I) as claimed in claim 1 wherein $R^1$ is selected from optionally substituted $C_{1-20}$ alkyl groups and $C_{6-20}$ aryl groups.

3. Compounds of formula (I) as claimed in claim 1 wherein m represents 1 in at least one of the groups $Y^1$, $Y^2$, $Y^3$ and $Y^4$; $R^2$ is a hydrogen atom; and $R^3$ is a hydrogen atom or a methyl group.

4. A pharmaceutical composition comprising as active ingredient one or more compounds of formula (I) as

defined in any preceding claim and/or a non-toxic salt thereof, together with a pharmaceutical carrier or excipient.

5. A process for the preparation of a compound of formula (I) as defined in any of claims 1 to 3, which comprises reaction of a compound of formula (II)

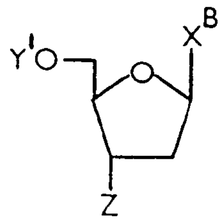

(II)

[wherein $Y^1$ and Z are as hereinbefore defined and $X^B$ is as hereinbefore defined for X except that any of the groups $Y^1$, $Y^2$, $Y^3$ and $Y^4$ may each additionaly represent a protecting group, with the proviso that at least one of $Y^1$, $Y^2$, $Y^3$ and $Y^4$ is a hydrogen atom] with a reagent serving to introduce a group $R^1(0)_n.CO.(OCR^2R^3)_m$- followed where required by removal of any protecting groups and/or unwanted substituents so introduced.

6. Use of compounds of formula (I) as defined in any of claims 1 to 3, and/or salts thereof, in the manufacture of a medicament for the treatment or prophylaxis of retrovirus infections.

**Claims for the following Contracting States : ES, GR**

1. A process for the preparation of compounds of formula (I)

(I)

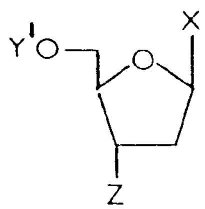

wherein Z is a hydrogen atom or an azido group,

$Y^1$ is a hydrogen atom or a physiologically acceptable group of the formula

$$R^1(O)_nCO(OCR^2R^3)_m-$$

where n is O or 1, m is 0 or 1 and

$R^1$ is an optionally substituted alkyl or aryl group or, where n is 0, a hydrogen atom;

$R^2$ and $R^3$ are independently hydrogen atoms or lower alkyl groups; and

X is a group selected from

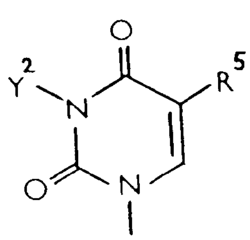

(A)

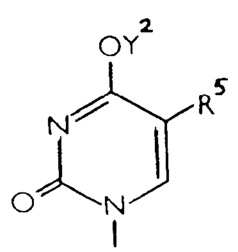

(B)

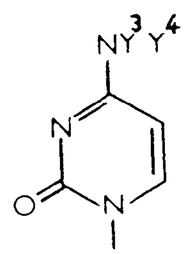

(C)

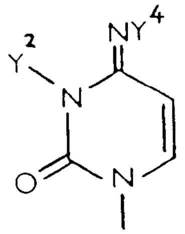
(D)

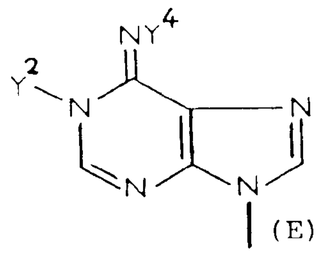
(E)

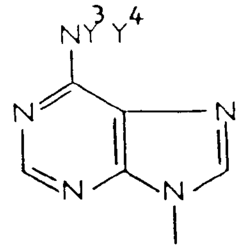
(F)

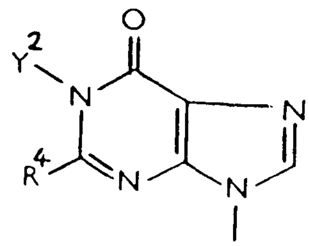
(G)

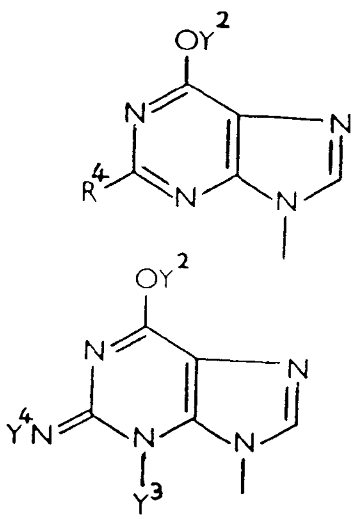
(H)

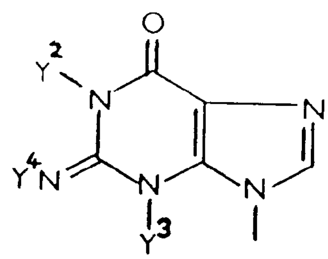
(I)

(J)

where the groups $Y^2$, $Y^3$ and $Y^4$ are as defined for $Y^1$ and may be the same as or different from $Y^1$ or each other, $R^4$ is a hydrogen atom or a group $-NY^3Y^4$, where $Y^3$ and $Y^4$ have the above meanings and $R^5$ is a hydrogen atom or a lower alkyl group, with the proviso that at least one of the groups $Y^1$, $Y^2$, $Y^3$ and $Y^4$ is a group of formula (III)

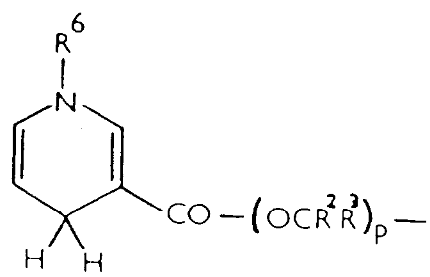

(III)

wherein $R^6$ is a $C_{1-7}$ alkyl group or $C_{7-10}$ aralkyl group, $R^2$ and $R^3$ are as defined above and p is O or 1, and that when $Y^1$ is a group of formula (III) as defined above, Z is an azide group and X is a group of formula (A) or (B) as defined above then $Y^2$ is a group of formula (III) or of formula $R^1(O)_nCO(OCR^3R^4)_m$- as defined above, which process comprises reaction of a compound of formula (II)

(II) 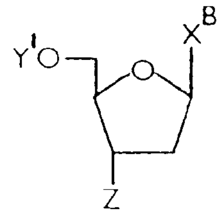

[wherein $Y^1$ and Z are as hereinbefore defined and $X^B$ is as hereinbefore defined for X except that any of the groups $Y^1$, $Y^2$, $Y^3$ and $Y^4$ may each additionaly represent a protecting group, with the proviso that at least one of $Y^1$, $Y^2$, $Y^3$ and $Y^4$ is a hydrogen atom] with a reagent serving to introduce a group $R^1(0)_n.CO.(OCR^2R^3)_m$- followed where required by removal of any protecting groups and/or unwanted substituents so introduced.

2. A process as claimed in claim 1 wherein $R^1$ is selected from optionally substituted $C_{1-20}$ alkyl groups and $C_{6-20}$ aryl groups.

3. A process as claimed in claim 1 wherein m represents 1 in at least one of the groups $Y^1$, $Y^2$, $Y^3$ and $Y^4$; $R^2$ is a hydrogen atom; and $R^3$ is a hydrogen atom or a methyl group.

4. Use of compounds of formula (I) as defined in any of claims 1 to 3, and/or salts thereof, in the manufacture of a medicament for the treatment or prophylaxis of retrovirus infections.

**Patentansprüche**

**Patentansprüche für folgende Vertragsstaaten : AT, BE, CH, DE, FR, GB, IT, LI, LU, NL SE**

1. Verbindungen der Formel (I)

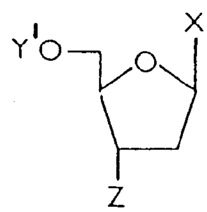 (I)

worin Z für ein Wasserstoffatom oder eine Azidogruppe steht,

$Y^1$ ein Wasserstoffatom oder eine physiologisch verträgliche Gruppe der Formel

$$R^1(0)_nC0(0CR^2R^3)_m-$$

bedeutet, worin n für 0 oder 1 steht, m für 0 oder 1 steht und

$R^1$ eine gegebenenfalls substituierte Alkyl- oder Arylgruppe ist oder, wenn n 0 ist, ein Wasserstoffatom bedeutet;

$R^2$ und $R^3$ unabhängig Wasserstoffatome oder Niedrigalkylgruppen sind; und

X eine Gruppe ist, ausgewählt unter:

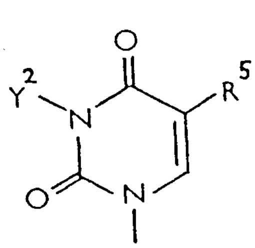
(A)

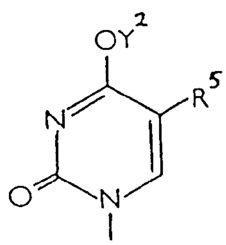
(B)

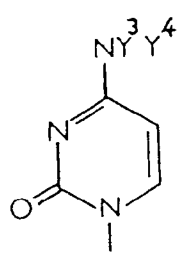
(C)

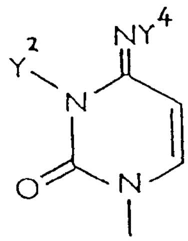 (D) 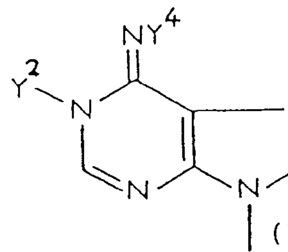 (E) 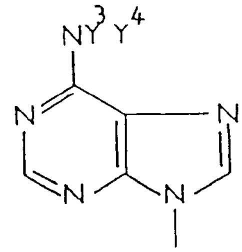 (F)

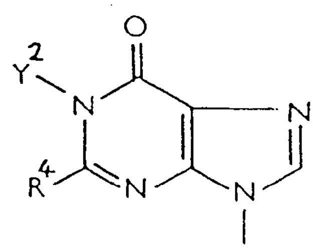 (G) 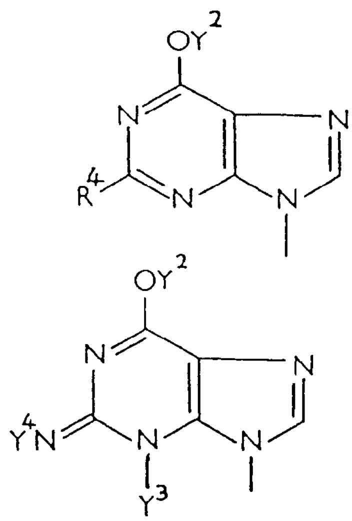 (H)

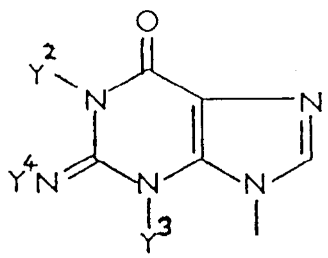 (I) (J)

worin die Gruppen $Y^2$, $Y^3$ und $Y^4$ die für $Y^1$ angegebene Bedeutung besitzen und die gleiche oder von $Y^1$ oder voneinander verschiedene Bedeutung haben können, $R^4$ ein Wasserstoffatom oder eine Gruppe $-NY^3Y^4$ darstellt, worin $Y^3$ und $Y^4$ die vorstehenden Bedeutungen besitzen und $R^5$ für ein Wasserstoffatom oder eine Niedrigalkylgruppe steht, mit der Maßgabe, daß zumindest eine der Gruppen $Y^1$, $Y^2$, $Y^3$ und $Y^4$ eine Gruppe der Formel (III)

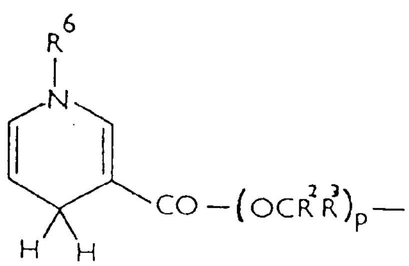 (III)

ist, worin $R^6$ eine $C_{1-7}$-Alkylgruppe oder $C_{7-10}$-Aralkylgruppe bedeutet, $R^2$ und $R^3$ wie vorstehend definiert sind und p für 0 oder 1 steht, und daß, wenn $Y^1$ eine Gruppe der Formel (III), wie vorstehend definiert, ist, Z eine Azidgruppe darstellt und X eine Gruppe der Formel (A) oder (B), wie vorstehend definiert, ist, dann $Y^2$ eine Gruppe der Formel (III) oder der Formel $R^1(0)_nC0(0CR^3R^4)_m$-, wie vorstehend definiert, darstellt.

2. Verbindungen der Formel (I) gemäß Anspruch 1, worin $R^1$ unter gegebenenfalls substituierten $C_{1-20}$-Alkylgruppen und $C_{6-20}$-Arylgruppen ausgewählt ist.

3. Verbindungen der Formel (I) gemäß Anspruch 1, worin m in zumindest einer der Gruppen $Y^1$, $Y^2$, $Y^3$ und $Y^4$ für 1 steht; $R^2$ ein Wasserstoffatom bedeutet; und $R^3$ ein Wasserstoffatom oder eine Methylgruppe darstellt.

4. Pharmazeutische Zusammensetzung, umfassend als Wirkstoff eine oder mehrere Verbindungen der Formel (I), wie in einem der vorhergehenden Ansprüche definiert, und/oder ein nicht-toxisches Salz hiervon zusammen mit einem pharmazeutischen Träger oder Exzipienten.

5. Verfahren zur Herstellung einer Verbindung der Formel (I), wie in einem der Ansprüche 1 bis 3 definiert, das die Umsetzung einer Verbindung der Formel (II)

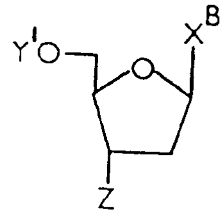

(II)

[worin $Y^1$ und Z wie vorstehend definiert sind und $X^B$ wie vorstehend für X definiert ist, mit Ausnahme dessen, daß jede der Gruppen $Y^1$, $Y^2$, $Y^3$ und $Y^4$ jeweils zusätzlich eine Schutzgruppe bedeuten kann, mit der Maßgabe, daß zumindest eine der Gruppen $Y^1$, $Y^2$, $Y^3$ und $Y^4$ ein Wasserstoffatom ist] mit einem Reagens, das der Einführung einer Gruppe $R^1(0)_n.C0.(0CR^2R^3)_m$- dient, umfaßt, woran sich erforderlichenfalls die Entfernung irgendwelcher Schutzgruppen und/oder so eingeführter, unerwünschter Substituenten anschließt.

6. Verwendung der Verbindungen der Formel (I), wie in einem der Ansprüche 1 bis 3 definiert, und/oder von deren Salzen bei der Herstellung eines Arzneimittels zur Behandlung oder Prophylaxe von Retrovirusinfektionen.

**Patentansprüche für folgende Vertragsstaaten : ES, GR**

1. Verfahren zur Herstellung von Verbindungen der Formel (I)

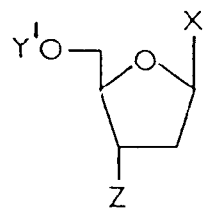

(I)

worin Z für ein Wasserstoffatom oder eine Azidogruppe steht,

$Y^1$ ein Wasserstoffatom oder eine physiologisch verträgliche Gruppe der Formel

$$R^1(0)_nC0(0CR^2R^3)_m -$$

bedeutet, worin n für 0 oder 1 steht, m für 0 oder 1 steht und

$R^1$ eine gegebenenfalls substituierte Alkyl- oder Arylgruppe ist oder, wenn n 0 ist, ein Wasserstoffatom bedeutet;

$R^2$ und $R^3$ unabhängig Wasserstoffatome oder Niedrigalkylgruppen sind; und

X eine Gruppe ist, ausgewählt unter:

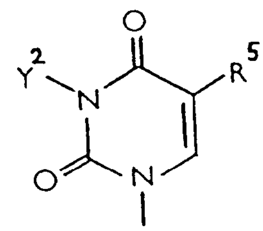
(A)

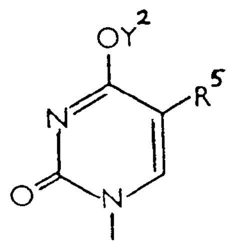
(B)

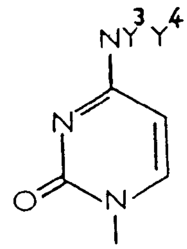
(C)

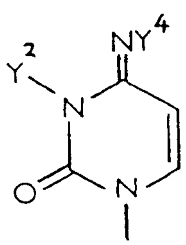
(D)

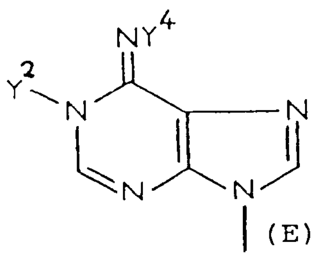
(E)

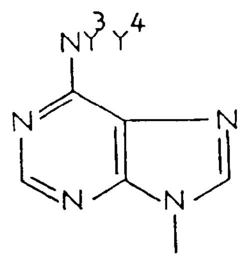
(F)

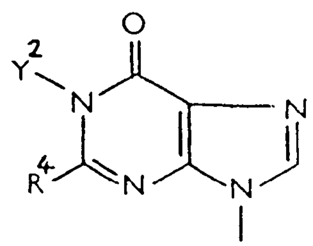
(G)

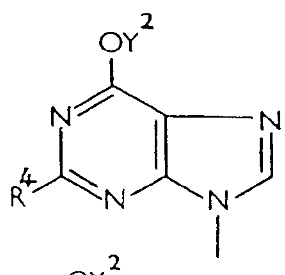
(H)

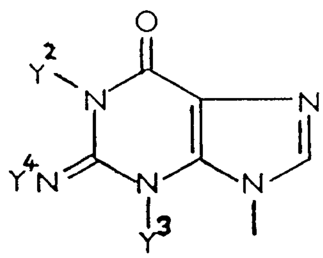
(I)

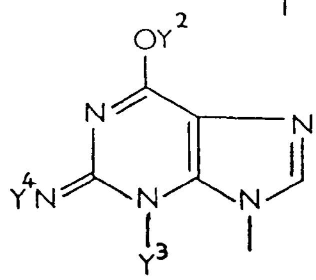
(J)

worin die Gruppen $Y^2$, $Y^3$ und $Y^4$ die für $Y^1$ angegebene Bedeutung besitzen und die gleiche oder von $Y^1$ oder voneinander verschiedene Bedeutung haben können, $R^4$ ein Wasserstoffatom oder eine Gruppe -$NY^3Y^4$ darstellt, worin $Y^3$ und $Y^4$ die vorstehenden Bedeutungen besitzen und $R^5$ für ein Wasserstoffatom oder eine Niedrigalkylgruppe steht, mit der Maßgabe, daß zumindest eine der Gruppen $Y^1$, $Y^2$, $Y^3$ und $Y^4$ eine Gruppe der Formel (III)

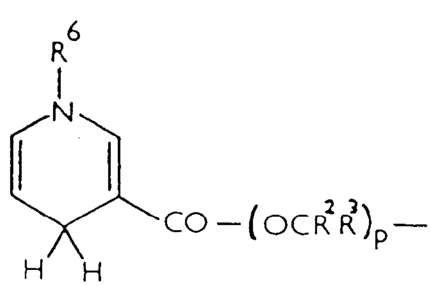
(III)

ist, worin $R^6$ eine $C_{1-7}$-Alkylgruppe oder $C_{7-10}$-Aralkylgruppe bedeutet, $R^2$ und $R^3$ wie vorstehend definiert sind

und p für 0 oder 1 steht, und daß, wenn $Y^1$ eine Gruppe der Formel (III), wie vorstehend definiert, ist, Z eine Azidgruppe darstellt und X eine Gruppe der Formel (A) oder (B), wie vorstehend definiert, ist, dann $Y^2$ eine Gruppe der Formel (III) oder der Formel $R^1(0)_nC0(0CR^3R^4)_m$-, wie vorstehend definiert, darstellt, welches Verfahren die Umsetzung einer Verbindung der Formel (II)

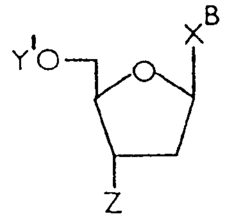

(II)

[worin $Y^1$ und Z wie vorstehend definiert sind und $X^B$ wie vorstehend für X definiert ist, mit Ausnahme dessen, daß jede der Gruppen $Y^1$, $Y^2$, $Y^3$ und $Y^4$ jeweils zusätzlich eine Schutzgruppe bedeuten kann, mit der Maßgabe, daß zumindest eine der Gruppen $Y^1$, $Y^2$, $Y^3$ und $Y^4$ ein Wasserstoffatom ist] mit einem Reagens, das der Einführung einer Gruppe $R^1(0)_n.C0.(0CR^2R^3)_m$- dient, umfaßt, woran sich erforderlichenfalls die Entfernung irgendwelcher Schutzgruppen und/oder so eingeführter, unerwünschter Substituenten anschließt.

2. Verfahren gemäß Anspruch 1, worin $R^1$ unter gegebenenfalls substituierten $C_{1-20}$-Alkylgruppen und $C_{6-20}$-Arylgruppen ausgewählt ist.

3. Verfahren gemäß Anspruch 1, worin m in zumindest einer der Gruppen $Y^1$, $Y^2$, $Y^3$ und $Y^4$ für 1 steht; $R^2$ ein Wasserstoffatom bedeutet; und $R^3$ ein Wasserstoffatom oder eine Methylgruppe darstellt.

4. Verwendung der Verbindungen der Formel (I), wie in einem der Ansprüche 1 bis 3 definiert, und/oder von deren Salzen bei der Herstellung eines Arzneimittels zur Behandlung oder Prophylaxe von Retrovirusinfektionen.

## Revendications

### Revendications pour les Etats contractants suivants : AT, BE, CH, DE, FR, GB, IT, LI, LU, NL, SE

1. Composés de formule (I) :

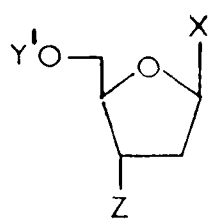

(I)

dans laquelle Z représente un atome d'hydrogène ou un groupe azido,

$Y^1$ représente un atome d'hydrogène ou un groupe physiologiquement acceptable de formule :

$$R^1(O)_nCO(OCR^2R^3)_m-$$

dans laquelle n est égal à 0 ou 1, m est égal à 0 ou 1, et

$R^1$ représente un groupe alkyle ou aryle éventuellement substitué, ou lorsque n est égal à 0, un atome d'hydrogène ;

$R^2$ et $R^3$ représentent indépendamment des atomes d'hydrogène ou des groupes alkyle inférieurs ; et

X représente un groupe choisi parmi

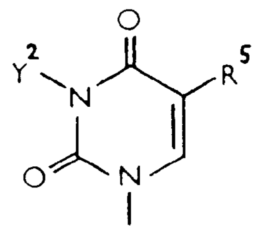
(A)
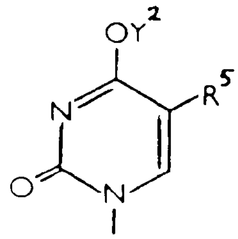
(B)
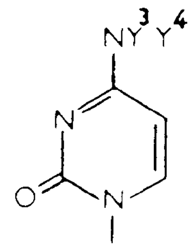
(C)

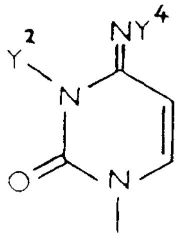
(D)
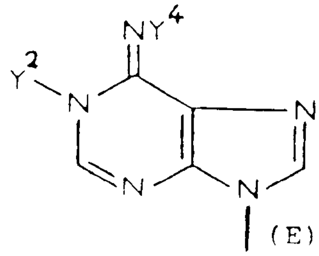
(E)
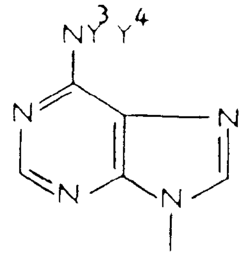
(F)

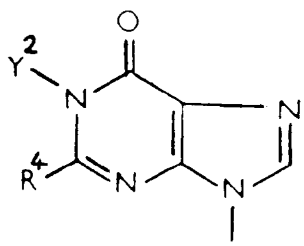
(G)
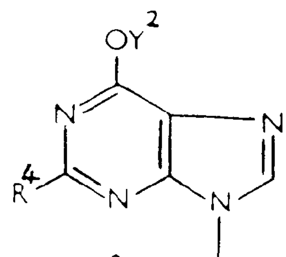
(H)

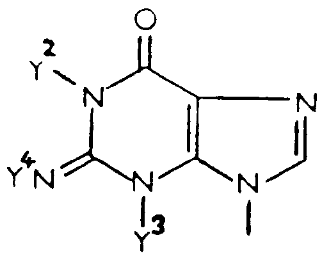
(I)
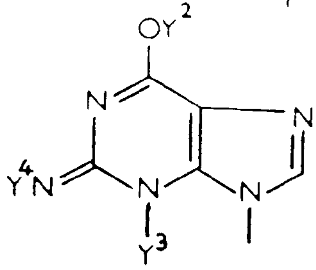
(J)

dans lesquels $Y^2$, $Y^3$ et $Y^4$ sont tels que définis pour $Y^1$, et ils peuvent être identiques ou différents de $Y^1$ ou entre eux, $R^4$ représente un atome d'hydrogène ou un groupe $-NY^3Y^4$, dans lequel $Y^3$ et $Y^4$ ont les mêmes définitions que ci-dessus, et $R^5$ représente un atome d'hydrogène ou un groupe alkyle inférieur, à condition qu'au moins l'un des groupes $Y^1$, $Y^2$, $Y^3$ et $Y^4$, représente un groupe de formule (III) :

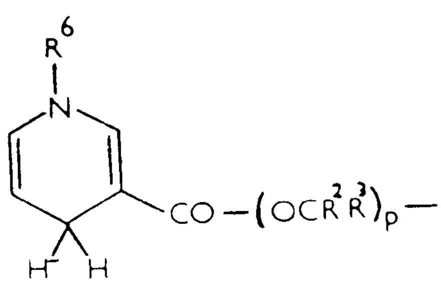
(III)

dans laquelle $R^6$ représente un groupe alkyle en $C_1$-$C_7$ ou un groupe aralkyle en $C_7$-$C_{10}$, et $R^2$ et $R^3$ sont tels que définis ci-dessus, et p est égal à 0 ou 1, et que, lorsque $Y^1$ représente un groupe de formule (III) que définie ci-dessus, Z représente un groupe azide et X un groupe de formule (A) ou (B) telle que définie ci-dessus, $Y^2$ représente alors un groupe de formule (III) ou de formule $R^1(O)_n(OCR^3R^4)_m$- telle que définie ci-dessus.

2. Composés de formule (I) selon la revendication 1, dans lesquels $R^1$ est choisi parmi les groupes alkyle en $C_1$-$C_{20}$ et aryle en $C_6$-$C_{20}$ éventuellement substitués.

3. Composés de formule (I) selon la revendication 1, dans lesquels m est égal à 1 dans au moins l'un des groupes $Y^1$, $Y^2$, $Y^3$ et $Y^4$ ; $R^2$ représente un atome d'hydrogène ; et $R^3$ représente un atome d'hydrogène ou un groupe méthyle.

4. Composition pharmaceutique comprenant comme ingrédient actif, un ou plusieurs des composés de formule (I) telle que définie dans l'une quelconque des revendications précédentes et/ou un sel non toxique de ceux-ci, avec un véhicule ou un excipient pharmaceutique.

5. Procédé de préparation d'un composé de formule (I) selon l'une quelconque des revendications 1 à 3, dans lequel on fait réagir un composé de formule (II) :

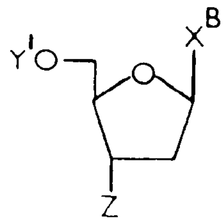

(II)

dans laquelle $Y^1$ et Z sont tels que définis ci-dessus, et $X^B$ est tel que défini ci-dessus pour X, à l'exception du fait que l'un des groupes $Y^1$, $Y^2$, $Y^3$ et $Y^4$ peut en outre représenter indépendamment un groupe protecteur, à condition qu'au moins l'un des groupes $Y^1$, $Y^2$, $Y^3$ et $Y^4$, représente un atome d'hydrogène ; avec un réactif servant à introduire un groupe $R^1(O)_n.CO.(OCR^2R^3)_m$-, puis lorsque cela est nécessaire, on élimine les groupes protecteurs et/ou les substituants non souhaitables ainsi introduits.

6. Utilisation des composés de formule (I) selon l'une quelconque des revendications 1 à 3, et/ou de sels de ceux-ci, pour la fabrication d'un médicament destiné au traitement ou à la prophylaxie d'infections rétrovirales.

**Revendications pour les Etats Contractants suivants : ES, GR**

1. Procédé de préparation de composés de formule (I) :

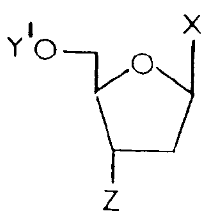

(I)

dans laquelle Z représente un atome d'hydrogène ou un groupe azido,

$Y^1$ représente un atome d'hydrogène ou un groupe physiologiquement acceptable de formule :

$$R^1(O)_nCO(OCR^2R^3)_m-$$

dans laquelle n est égal à 0 ou 1, m est égal à 0 ou 1, et

$R^1$ représente un groupe alkyle ou aryle éventuellement substitué, ou lorsque n est égal à 0, un atome d'hydrogène ;

$R^2$ et $R^3$ représentent indépendamment des atomes d'hydrogène ou des groupes alkyle inférieurs ; et

X représente un groupe choisi parmi les groupes :

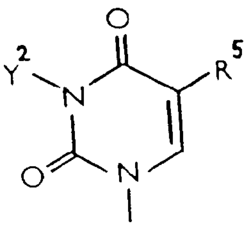
(A)

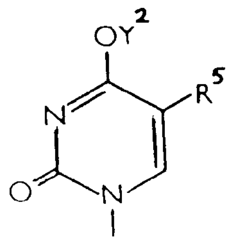
(B)

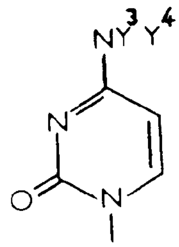
(C)

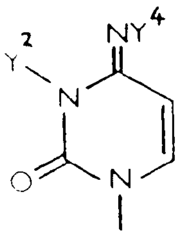
(D)

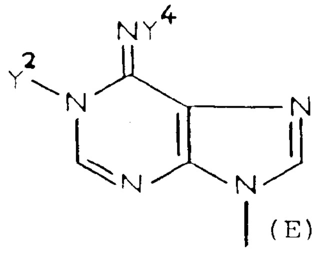
(E)

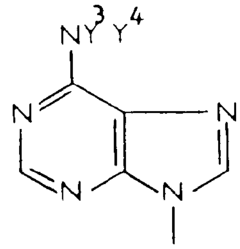
(F)

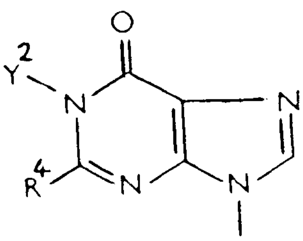
(G)

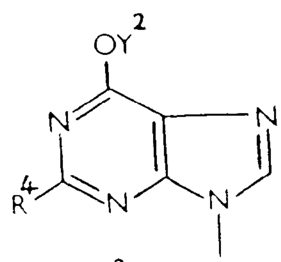
(H)

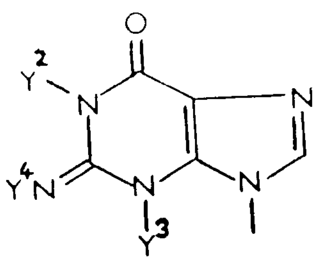
(I)

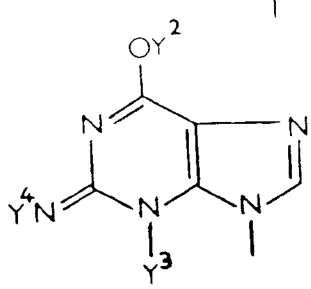
(J)

dans lesquels les groupes $Y^2$, $Y^3$ et $Y^4$ sont tels que définis ci-dessus pour $Y^1$, et ils peuvent être identiques ou différents de $Y^1$ ou entre eux, $R^4$ représente un atome d'hydrogène ou un groupe $-NY^3Y^4$, dans lequel $Y^3$ et $Y^4$ ont les mêmes définitions que ci-dessus, et $R^5$ représente un atome d'hydrogène ou un groupe alkyle inférieur, à condition qu'au moins l'un des groupes $Y^1$, $Y^2$, $Y^3$ et $Y^4$, représente un groupe de formule (III) :

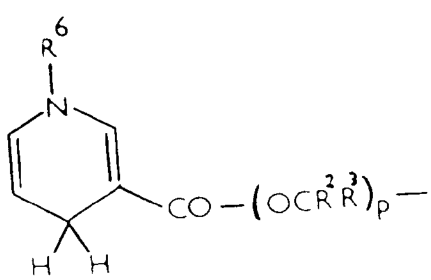
(III)

dans laquelle $R^6$ représente un groupe alkyle en $C_1$-$C_7$ ou aralkyle en $C_7$-$C_{10}$, et $R^2$ et $R^3$ sont tels que définis ci-dessus, et p est égal à 0 ou 1, et que, lorsque $Y^1$ représente un groupe de formule (III) telle que définie ci-dessus, Z représente un groupe azide et X un groupe de formule (A) ou (B) telle que définie ci-dessus, $Y^2$ représente alors un groupe de formule (III) ou de formule $R^1(O)_nCO\ (OCR^3R^4)_m$- telle que définie ci-dessus, selon lequel on fait réagir un composé de formule (II) :

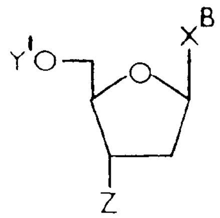

(II)

(dans laquelle $Y^1$ et Z sont tels que définis ci-dessus, et $X^B$ est tel que défini ci-dessus pour X, à l'exception du fait que l'un des groupes $Y^1$, $Y^2$, $Y^3$ et $Y^4$ peut en outre représenter indépendamment un groupe protecteur, à condition qu'au moins l'un des groupes $Y^1$, $Y^2$, $Y^3$ et $Y^4$ représente un atome d'hydrogène) ; avec un réactif servant à introduire un groupe $R^1(O)_n.CO.(OCR^2R^3)_m$-, puis si cela est nécessaire, on élimine les groupes protecteurs et/ou les substituants non souhaitables ainsi introduits.

2. Procédé selon la revendication 1, dans lequel $R^1$ est choisi parmi les groupes alkyle en $C_1$-$C_{20}$ et aryle en $C_6$-$C_{20}$ éventuellement substitués.

3. Procédé selon la revendication 1, dans lequel m représente 1 dans au moins l'un des groupes $Y^1$, $Y^2$, $Y^3$ et $Y^4$ ; $R^2$ représente un atome d'hydrogène ; et $R^3$ représente un atome d'hydrogène ou un groupe méthyle.

4. Utilisation de composés de formule (I) selon l'une quelconque des revendications 1 à 3, et/ou de sels de ceux-ci, pour la fabrication d'un médicament destiné au traitement ou à la prophylaxie d'infections rétrovirales.